Europäisches Patentamt

European Patent Office

Office européen des brevets

(19)

(11) **EP 1 312 358 A1**

(12) **EUROPEAN PATENT APPLICATION**

(43) Date of publication:
**21.05.2003 Bulletin 2003/21**

(51) Int Cl.⁷: **A61K 9/16**, A61K 31/496

(21) Application number: **02257581.5**

(22) Date of filing: **01.11.2002**

(84) Designated Contracting States:
**AT BE BG CH CY CZ DE DK EE ES FI FR GB GR
IE IT LI LU MC NL PT SE SK TR**
Designated Extension States:
**AL LT LV MK RO SI**

(30) Priority: **16.11.2001 CA 2363376**

(71) Applicant: **SHERMAN, Bernard Charles
Willowdale, Ontario M2L 2K1 (CA)**

(72) Inventor: **SHERMAN, Bernard Charles
Willowdale, Ontario M2L 2K1 (CA)**

(74) Representative: **Howard, Paul Nicholas et al
Carpmaels & Ransford
43 Bloomsbury Square
London WC1A 2RA (GB)**

(54) **Solid pharmaceutical compositions for oral administration comprising itraconazole**

(57)    Co-precipitates of itraconazole and a water-soluble non-polymeric compound. Compositions for oral administration comprising amorphous itraconazole or a co-precipitate, and a disintegrant.

EP 1 312 358 A1

Printed by Jouve, 75001 PARIS (FR)

**Description**

<u>BACKGROUND OF THE INVENTION</u>

Itraconazole is an antifungal compound that is therapeutically useful when appropriately administered either topically or orally. It is a mixture of stereoisomers, and the term "itraconazole" when used herein will be understood to include individual stereoisomers and mixtures thereof.

The development of itraconazole compositions for oral administration, which will maximize absorption after ingestion, has been made difficult by the fact that itraconazole is only very sparingly soluble in water. There are several prior art approaches for overcoming the low level of solubility.

U.S. Patent No. 5,707,975 discloses that the solubility and thus also the bioavailability (i.e. extent of absorption upon oral administration) of itraconazole can be increased by complexation with cyclodextrins or derivatives thereof. An aqueous solution for oral administration comprising itraconazole complexed with a cyclodextrin, apparently made in accordance with the teaching of this patent, is sold in the United States and elsewhere under the tradename Sporanox™. There is no solid composition for oral administration (i.e. tablets or capsules) using this technology.

U.S. patent 5,633,015 discloses two-piece hard gelatin capsules for oral administration. The capsules contain a multitude of small beads, wherein the beads comprise inert cores, which are coated with a film coating of itraconazole and a hydrophilic polymer. The coating is applied by dissolving the itraconazole and polymer in organic solvent and spraying the solution onto the cores in a fluid bed dryer; the solvent is evaporated to dry the coating as it is being applied. The polymer serves to cause the coating to adhere to the surface of the beads.

It appears that this approach improves the dissolution and absorption of the itraconazole through a combination of several effects as follows:

> i) pure itraconazole is usually crystalline. However, when the solution of itraconazole and polymer is applied to the beads, the solvent evaporates too rapidly to allow the itraconazole to precipitate as crystals; the film that forms on the surface of the beads is thus an amorphous co-precipitate of the itraconazole and polymer. Substances in amorphous (i.e. non-crystalline) form will generally dissolve much more rapidly than crystalline form;

> ii) the hydrophilic polymer is more water-soluble than itraconazole, so that the rate of dissolution of the film coating (i.e. the co-precipitate) is greater than that of pure itraconazole; and

> iii) the coating is spread over the relatively large surface area of the many beads, and this large surface area also facilitates more rapid dissolution.

Capsules for oral administration made in accordance with the teaching of U.S. Patent No. 5,633,015, in strength of 100 mg, are sold in the United States and elsewhere under the tradename Sporanox™.

The composition of Sporanox™ capsules is still less than ideal because:

> i) the process of making the beads (i.e. making the cores and applying the film coating) is relatively complex; and

> ii) the amount of itraconazole in the composition is under 25% of the total weight of the beads. This means that capsules comprising 100 mg of itraconazole must have a fill weight of over 400 mg. This requires use of size 0 capsules, which are relatively large.

U.S. Patent No. 5,776,495 discloses and claims a co-precipitate of a therapeutic agent (which may be itraconazole) and a hydrophilic polymer, obtained by dissolving the therapeutic agent and polymer in organic solvent and evaporating the solvent to form a co-precipitate. No example is given using itraconazole as the therapeutic agent. The process of this patent is similar to that of U.S. Patent 5,633,015, except only that in U.S. Patent 6,027,747, the solvent is evaporated to form the co-precipitate as a solid foam which can be ground into small particles, whereas in U.S. Patent 5,633,015, the co-precipitate is deposited as a film on the surface of the beads.

International Publication No. WO 98/57967 discloses compositions having improved bioavailability (i.e. absorption) comprising itraconazole in the form of small (0.5 to 10 micron) amorphous particles which are obtained by "dissolution-induced" drying, by which is meant preparing a solution of itraconazole in organic solvent and rapidly evaporating the solvent so as not to enable crystalline formulation. The itraconazole thus precipitates as small amorphous particles.

The approach in WO 98/57967 is similar to that in U.S. Patent Nos. 5,633,015 and 6,027,747 in that, in all three cases, the itraconazole is converted to an amorphous precipitate. The differences among these references are only as to whether the precipitate is pure itraconazole (as in WO98/57967) or a co-precipitate with a hydrophilic polymer (as in U.S. Patent Nos. 5,633,015 and 6,027,747).

While WO98/57967 avoids use of the hydrophilic polymer, to give pure amorphous itraconazole, there are still two problems as follows:

> i) to obtain small particles, it is necessary to spray-dry from dilute solutions, so that relatively large amounts of solvent must be used; and

ii) the amorphous particles tend to agglomerate into large particles, which dissolve only slowly. To overcome this problem, the amorphous itraconazole powder must be diluted with a relatively large amount of excipients (i.e. inactive ingredients). The final mixture is thus again less than 25% itraconazole by weight, so that a relatively large (size 0) capsule is still needed to contain 100 mg of itraconazole.

U.S. Patent Application No. 2001/0007678 discloses solid dispersions of itraconazole in a water-soluble polymer. The resulting dispersions are similar to the "co-precipitates" with hydrophilic polymer disclosed in U.S. Patent Nos. 5,633,015 and 6,027,747. U.S. Patent Application No. 2001/0007678 discloses that such solid dispersions can be obtained by dispersing the itraconazole in polymer in a hot molten state, solidifying the molten mix, grinding the resultant solid into particles, and further processing the particles into tablets or capsules. The processes disclosed are again relatively complex and require specialized equipment.

U.S. Patent No. 6,039,981 discloses a solid composition comprising a fused mixture of itraconazole and phosphoric acid. While itraconazole has a melting point of about 170°C, the melting point of phosphoric acid is only 42°C. This patent discloses that the fused mixture can be obtained by mixing the itraconazole and phosphoric acid, heating the mixture to a temperature of from 100 to 170°C to obtain a homogenous melt, adding a carrier and surfactant, cooling the resulting solid, and pulverizing the solid. The powder is then further processed into capsules or tablets.

While such a composition again enables good dissolution and absorption, the process of manufacture is again relatively complex. Also, because of the low melting point of the phosphoric acid, the resulting fused mixture is tacky, making it difficult to further process the fused mixture, unless the mixture is diluted with a relatively large amount of non-tacky excipients, which increases the required size of the final capsules or tablets. U.S. Patent No. 5,750,147 discloses microspheres, which exhibit improved dissolution. The microspheres comprise itraconazole and a microsphere-forming carrier and are said to enable high bioavailability. However, the quantities of microsphere-forming carrier are relatively large, so that again large capsules would be required to comprise 100 mg of itraconazole.

In light of the prior art, the object of the present invention is to enable solid compositions (i.e. capsules or tablets) for oral administration that comprise itraconazole, that provide for relatively rapid dissolution in gastro-intestinal fluid after ingestion (and thus high extent of absorption), that can be made by relatively simple processes, and that enable capsules of 100 mg strength that are relatively small (i.e. smaller than size 0).

## BRIEF SUMMARY OF THE INVENTION

One aspect of the invention is co-precipitate of itraconazole and a water-soluble non-polymeric compound.
A second aspect of the invention is compositions for oral administration comprising amorphous itraconazole or a co-precipitate of itraconazole, in admixture with a disintegrant.

## DETAILED DESCRIPTION OF THE INVENTION

The term "co-precipitate" is to be understood to mean an amorphous (non-crystalline) solid substance comprising two or more compounds that is obtained by dissolving the compounds in solvent (i.e. a single solvent or mixture of solvents) in which the compounds are soluble and evaporating the solvent sufficiently quickly so that the compounds precipitate together and not as separate crystals or particles of the individual substances.
The evaporation of the solvent may be done by any suitable means, including for example, while mixing in a rotary evaporator under vacuum, or by spray-drying the solution. Spray drying may be done in a spray dryer, or alternatively in a fluid-bed dryer. Alternatively, the solution may be mixed into or sprayed onto other excipients, and the resultant wet mixture then dried.
As aforesaid, the co-precipitates of the present invention comprise itraconazole and a water-soluble non-polymeric compound. The inclusion of a water-soluble compound that is non-polymeric serves to increase the dissolution rate of the co-precipitate relative to the dissolution rate of pure amorphous itraconazole and relative to a co-precipitate of itraconazole with a water-soluble polymer. It thus becomes possible to use a co-precipitate of relating large particle size, which can be prepared without the use of relatively large amount of organic solvent. The water-soluble non-polymeric compound will preferably have a melting point above 50°C, and more preferably above 100°C, to render the co-precipitate non-tacky for easier subsequent processing. The water-soluble non-polymeric compound may be any water-soluble non-polymeric compound that is acceptable (by reason of its lack of toxicity) for use as an excipient (i.e. inactive ingredient) in pharmaceutical products for oral administration. The water-soluble non-polymeric compound will preferably have a solubility in water at 20°C greater than 1 part per 30 parts water by weight, more preferably greater than 1 part per 10 parts water, and even more preferably greater than 1 part per 3 parts water.
Suitable compounds for use as the water-soluble non-polymeric compound will include, for example, sucrose, lactose, dextrose, mannitol and sorbitol. The water-soluble non-polymeric compound will preferably be an acid such as, for example, citric acid, malic acid, maleic acid, tartaric acid or sodium bisulfate. Especially preferred are tartaric acid and sodium bisulfate.

The amount of the water-soluble non-polymeric compound by weight will be preferably between 0.02 part and 2 parts per part itraconazole, more preferably between 0.05 part and 1 part per part itraconazole, and most preferably between 0.1 part and 0.5 part per part itraconazole.

As aforesaid, a co-precipitate of the invention may be made by dissolving the itraconazole and the water-soluble non-polymeric compound in solvent and evaporating the solvent quickly, so that the compounds precipitate together and not as separate crystals or particles of the individual substances. The solvent may comprise one or more organic solvents (such as, for example, chlorinated hydrocarbons or lower alcohols) and may also comprise water and acids.

As aforesaid, a second aspect of the invention is compositions for oral administration comprising amorphous itraconazole or a co-precipitate of itraconazole and a water-soluble compound, in admixture with a disintegrant.

When a co-precipitate is used, it will preferably be a co-precipitate according to the present invention as described *supra.*

While the dissolution rate of amorphous itraconazole or a co-precipitate of itraconazole and a water-soluble compound exceeds the dissolution rate of crystalline itraconazole, all such materials still exhibit poor dissolution in undiluted form. This is a result of the fact that, when added to gastrointestinal fluid or other aqueous medium, they tend to agglomerate rather than disperse.

It has been found that rapid dissolution in gastrointestinal fluid can be achieved by adequately diluting (i.e. mixing) the amorphous itraconazole or co-precipitate with suitable excipients before further processing into dosage forms such as tablets or capsules.

Such excipients will preferably comprise disintegrants, which will be understood to mean substances that are insoluble in water, but absorb water and swell, thus causing disintegration. Suitable disintegrants include, for example, croscarmellose sodium, carboxymethylcellulose calcium, sodium starch glycolate and crospovidone.

Such excipients may also include substances other than disintegrants such as, for example, a lubricant, such as magnesium stearate or stearic acid, or a glidant such as colloidal silicon dioxide. However, the excipients other than the disintegrant will preferably be minimal. The disintegrant will preferably constitute more than 40% by weight of the mixture, more preferably more than 45%, even more preferably more than 50%, and most preferably from 55% to 80%.

The disintegrant will preferably be either croscarmellose sodium or carboxymethylcellulose calcium.

Compositions of the present invention enable capsules comprising 100 mg of itraconazole and having good bioavailability to be in size 1 or size 2 capsules, which are smaller than size 0 capsules otherwise required.

The invention will be further understood from the following examples, which are intended to be illustrative and not limiting.

### Example 1

The following ingredients were added to a flask of suitable size in the proportions shown, and mixed until a clear solution resulted:

| Itraconazole | 100. |
|---|---|
| Tartaric acid NF | 20. |
| Methylene chloride NF | 500. |
| Methanol NF | 130. |
| | 750. |

The solution was spray-dried to produce a co-precipitate comprising itraconazole and tartaric acid in a ratio of 20 parts tartaric acid to 100 parts itraconazole.

### Example 2

Croscarmellose sodium was mixed with the co-precipitate of example 1, in the proportion as follows:

| Co-precipitate of example 1 | 120. |
|---|---|
| Croscarmellose sodium NF | 170. |
| | 290. |

### Example 3

The powder mixture of example 2 was filled into size 2 two-piece hard gelatin capsules at a net fill of 290 mg per capsule. Each capsule thus comprised 100 mg of itraconazole.

The dissolution rate of the capsules of this example was tested in United States Pharmacopoeia dissolution apparatus No. 2 at 75 rpm. The dissolution media was 900 ml of 0.1 N hydrochloric acid.

The amount dissolved was found to average over 80% in 1 hour, which is similar to the results achieved with Sporanox™ capsules 100 mg.

## Claims

1. A co-precipitate of itraconazole and a water-soluble non-polymeric compound, wherein the said compound has melting a point above 50°C and solubility exceeding 1 part per 30 parts water at 20°C.

2. A co-precipitate of claim 1 wherein the said compound has a solubility exceeding 1 part per 10 parts water at 20°C.

3. A co-precipitate of claim 1 wherein the said compound has solubility exceeding 1 part per 3 parts water at 20°C.

4. A co-precipitate of any of claims 1 to 3 wherein the melting point of the said compound is above 100°C.

5. A co-precipitate of any of claims 1 to 4 wherein the said compound is selected from the group consisting of lactose, sucrose, dextrose, mannitol and sorbitol.

6. A co-precipitate of any of claims 1 to 4 wherein the said compound is a non-volatile acid.

7. A co-precipitate of claim 6 wherein the said compound is citric acid.

8. A co-precipitate of claim 6 wherein the said compound is malic acid.

9. A co-precipitate of claim 6 wherein the said compound is maleic acid.

10. A co-precipitate of claim 6 wherein the said compound is tartaric acid.

11. A co-precipitate of claim 6 wherein the said compound is sodium bisulfate.

12. A co-precipitate of any of claims 1 to 11 wherein the amount of said compound is between 0.02 parts and 2 parts per part itraconazole by weight.

13. A co-precipitate of any of claims 1 to 11 wherein the amount of such compound is between 0.05 part and 1 part per part itraconazole by weight.

14. A co-precipitate of any of claims 1 to 11 wherein the amount of such compound is between 0.1 part and 0.5 part per part itraconazole by weight.

15. A solid composition for oral administration comprising a co-precipitate of any of claims 1 to 14.

16. A solid composition for oral administration which comprises a mixture of i) amorphous itraconazole or a co-precipitate of itraconazole and a water-soluble compound, and ii) a disintegrant, wherein the amount of disintegrant is greater than 40% of the mixture by weight.

17. A composition of claim 16, which comprises a co-precipitate of itraconazole and a water-soluble compound, wherein said co-precipitate is a co-precipitate of any of claims 1 to 14.

18. A composition of claim 16 or 17 wherein the amount of disintegrant is more than 45% of the mixture by weight.

19. A composition of claim 18 wherein the amount of disintegrant is more than 50% of the mixture by weight.

20. A composition of claim 19 wherein the amount of disintegrant is from 55% to 80% of the mixture by weight.

21. A composition of any of claims 16 to 20 wherein the disintegrant comprises croscarmellose sodium.

22. A composition of any of claims 16 to 20 wherein the disintegrant comprises carboxymethylcellulose calcium.

23. A capsule of size smaller than size 0 containing 100 mg of itraconazole in the form of a composition of any of claims 15 to 22.

24. A capsule of size 1 contains 100 mg of itraconazole in the form of a composition of any of claims 15 to 22.

25. A capsule of size 2 contains 100 mg of itraconazole in the form of a composition of any of claims 15 to 22.

**European Patent Office**

**EUROPEAN SEARCH REPORT**

Application Number

EP 02 25 7581

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 01 51029 A (ETHYPHARM LAB PROD ETHIQUES ;SUPLIE PASCAL (FR); GUERIN EMMANUEL ()) 19 July 2001 (2001-07-19) * page 1, line 4 - line 9 * * claims 1,4,7,8 * * page 1, line 26 - line 30 * * page 8, line 16 * * page 9, line 4 - line 14 * * page 11 - page 12; examples 1-3 * | 1-10, 12-15 | A61K9/16 A61K31/496 |
| X | WO 98 55148 A (JANSSEN PHARMACEUTICA NV ;VANDECRUYS ROGER PETRUS GEREBE (BE)) 10 December 1998 (1998-12-10) * page 18 - page 19; example 1 * * page 24, line 5 - line 10 * | 1-4, 6-10,15, 23-25 | |
| Y,D | WO 98 57967 A (CHA BONG JIN ;KIM SU EON (KR); OH JUN GYO (KR); DONG A PHARMACEUTI) 23 December 1998 (1998-12-23) * page 1, line 12 - line 14 * * page 2, line 14 - line 16 * * page 4, line 19 - page 5, line 5 * * page 6-8; examples 1-5 * | 16-25 | TECHNICAL FIELDS SEARCHED (Int.Cl.7) A61K |
| P,X | WO 01 97853 A (BIOCHEMIE GMBH ;BHARATRAJAN RAMASWAMI (IN); HEGDE DEEPAK (IN); NER) 27 December 2001 (2001-12-27) * page 1, line 7 - line 9 * * page 3, line 6 - line 8 * * page 11; examples 5,6 * | 1-4,6, 12-15, 23-25 | |
| X | CHOWDARY K P R: DRUG DEVELOPMENT AND INDUSTRIAL PHARMACY, vol. 26, no. 11, 2000, pages 1207-1211, XP008013501 | 16-25 | |
| Y | * page 1208, column 1, paragraph 3 - page 1210, column 1, paragraph 1 * | 16-25 | |

The present search report has been drawn up for all claims

| Place of search | Date of completion of the search | Examiner |
|---|---|---|
| THE HAGUE | 10 February 2003 | VON EGGELKRAUT, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 02 25 7581

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2003

| Patent document cited in search report | | Publication date | Patent family member(s) | | Publication date |
|---|---|---|---|---|---|
| WO 0151029 | A | 19-07-2001 | FR | 2803748 A1 | 20-07-2001 |
| | | | AU | 3189501 A | 24-07-2001 |
| | | | WO | 0151029 A1 | 19-07-2001 |
| WO 9855148 | A | 10-12-1998 | AU | 8108198 A | 21-12-1998 |
| | | | CN | 1258220 T | 28-06-2000 |
| | | | WO | 9855148 A1 | 10-12-1998 |
| | | | EP | 0998304 A1 | 10-05-2000 |
| | | | HU | 0004924 A2 | 28-08-2001 |
| | | | JP | 2002511073 T | 09-04-2002 |
| | | | NO | 995925 A | 11-01-2000 |
| | | | US | 2002150616 A1 | 17-10-2002 |
| | | | ZA | 9804849 A | 06-12-1999 |
| WO 9857967 | A | 23-12-1998 | AT | 216699 T | 15-05-2002 |
| | | | AU | 7939298 A | 04-01-1999 |
| | | | BR | 9810124 A | 08-08-2000 |
| | | | CN | 1262682 T | 09-08-2000 |
| | | | DE | 69805070 D1 | 29-05-2002 |
| | | | DE | 69805070 T2 | 07-11-2002 |
| | | | DK | 991646 T3 | 10-06-2002 |
| | | | EP | 0991646 A1 | 12-04-2000 |
| | | | ES | 2177022 T3 | 01-12-2002 |
| | | | HU | 0004050 A2 | 28-06-2001 |
| | | | JP | 2002504930 T | 12-02-2002 |
| | | | WO | 9857967 A1 | 23-12-1998 |
| | | | PL | 338631 A1 | 06-11-2000 |
| | | | PT | 991646 T | 30-08-2002 |
| | | | US | 6346533 B1 | 12-02-2002 |
| WO 0197853 | A | 27-12-2001 | AU | 6608101 A | 02-01-2002 |
| | | | WO | 0197853 A1 | 27-12-2001 |

EPO FORM P0459

For more details about this annex : see Official Journal of the European Patent Office, No. 12/82